# EUROPEAN PATENT APPLICATION

(11) **EP 1 481 678 A1**
(43) Date of publication of application: **01.12.2004**
(21) Application number: 03743594.8
(22) Date of filing: 04.03.2003
(51) Int. Cl.: A61K 31/403, A61K 31/404, A61K 31/427, A61K 31/47, A61K 31/502, A61K 31/517, A61K 45/00, A61P 35/00, A61P 43/00

(54) **ANTITUMOR AGENT COMPRISING COMBINATION OF SULFONAMIDE-CONTAINING HETEROCYCLIC COMPOUND WITH ANGIOGENESIS INHIBITOR**

(30) Priority: 05.03.2002 JP 2002059471
(71) Applicant: Eisai Co., Ltd., Tokyo 112-8088 (JP)
(72) Inventor: WAKABAYASHI, Toshiaki, Tsukuba-shi, Ibaraki 305-0042 (JP); ONO, Naoto, Ushiku-shi, Ibaraki 300-1222 (JP); SEMBA, Taro, Ushiku-shi, Ibaraki 300-1207 (JP); HANEDA, Toru, Ushiku-shi, Ibaraki 300-1216 (JP)
(74) Representative: HOFFMANN EITLE
(86) International application number: PCT/JP2003/002492
(87) International publication number: WO 2003/074045

(57) **Abstract**

The present invention provides a composition and a kit for treating tumors, which permits a sulfonamide-containing heterocyclic compound to exhibit its angiogenesis inhibitory activity and antitumor activity more effectively. According to the present invention, the sulfonamide-containing heterocyclic compound can be used in treating cancers more effectively by combination with a VEGF inhibitor/FGF inhibitor.

## Description

### Technical Field

The present invention relates to an antitumor agent and a kit for treating tumors, which comprises a sulfonamide-containing heterocyclic compound inhibiting expression of integrin and inhibiting angiogenesis, and an angiogenesis inhibitor, preferably a VEGF inhibitor or FGF inhibitor.

### Prior Art

Chemicals used conventionally as cancer chemotherapy act cytocidally on cancer cells. The chemicals include an alkylating agent such as cyclophosphamide, antimetabolites such as methotrexate and fluorouracil, antibiotics such as adriamycin, mitomycin and bleomycin, plant-derived agents such as taxol, vincristine and ethoposide, and a metal complex such as cisplatin. However, the conventional antitumor agents having a cytocidal mechanism of action are poor in antitumor effect, and there has been demand for development of antitumor agents based on a new mechanism action.

### Disclosure of the Invention

To meet this demand, the present inventors found that novel sulfonamide-containing heterocyclic compounds (WO 01/47891) exhibit an inhibitory action on angiogenesis, and further exhibit an antitumor effect in nude mice model. Among the compounds, E7820 represented by the following formula (II) exhibited a particularly strong inhibitory action on angiogenesis, and exhibited an activity on various types of cancers.

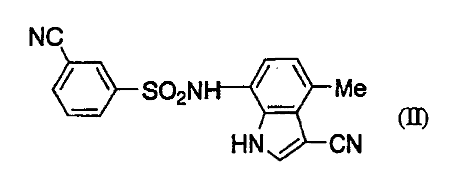

The object of the invention is to find a composition and a kit for treating tumors, which allows a sulfonamide-containing heterocyclic compound to exhibit its angiogenesis inhibitory activity and antitumor activity more effectively.

It is known that a process of angiogenesis is not only a phenomenon such as endothelial cell growth, cell migration and lumen formation in which endothelial cells alone are involved but is also a phenomenon in which other cells such as mast cells, fibroblasts and smooth muscle cells are involved, and it is reported that various angiogenesis factors such as VEGF (vascular endothelial growth factor), bFGF (basic fibroblast growth factor) and angiopoietin, matrix metalloprotease, and integrin are important in the process of angiogenesis. A plurality of cell species and a plurality of factors are involved in the process of angiogenesis, and thus the present inventors anticipated that a plurality of angiogenesis inhibitors different in site of action can be combined to achieve a more effective inhibitory action on angiogenesis than by administering a single component.

The present inventors have reported that sulfonamide-containing heterocyclic compounds inhibit expressionof integrin α2 to inhibit angiogenesis (WO 01/56607) . The present inventors made extensive study in searching for a composition which permits the sulfonamide-containing heterocyclic compound to exhibit its angiogenesis inhibitory activityandantitumoractivitymoreeffectively, andasaresult they found that the sulfonamide-containing heterocyclic compound is used in combination with a VEGF inhibitor or FGF inhibitor thereby exhibiting a synergistic effect on angiogenesis and further exhibiting an excellent antitumor activity.

The sulfonamide-containing heterocyclic compound exhibits a synergistic effect not only with inhibitors of VEGF receptor kinase/FGF receptor kinase but also with VEGF antibody/FGF antibody having a different working site, and a high synergistic effect can be expected not only by the VEGF inhibitor/FGF inhibitor shown in the Examples in this specification but also by VEGF inhibitors/FGF inhibitors on the whole.

That is, the present invention relates to:
1) an antitumor agent comprising a sulfonamide-containing heterocyclic compound represented by the formula (I^{a}) : 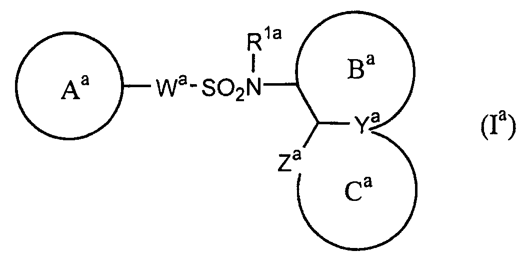 (wherein, the ring A^{a} represents an optionally substituted monocyclic or bicyclic aromatic ring; the ring B^{a} represents a 6-membered unsaturated hydrocarbon or a unsaturated 6-membered heterocyclic ring containing one nitrogen atom as a heteroatom, each of which may be substituted; the ring C^{a} represents an optionally substituted 5-membered heterocyclic ring containing one or two nitrogen atoms; R^{1a} represents a hydrogen atom or an alkyl group having one to six carbon atoms; W^{a} represents a single bond or -CH=CH-; Y^{a} represents a carbon atom or a nitrogen atom; and Z^{a} represents -N(R^{2a})- (wherein R^{2a} represents a hydrogen atomor a lower alkyl group) or a nitrogen atom) , a pharmacologically acceptable salt thereof or a hydrate of them combined with an angiogenesis inhibitor;
2) the antitumor agent described in 1), wherein W^{a} is a single bond;
3) the antitumor agent described in 1), wherein W^{a} is a single bond, Z^{a} is -NH- and Y^{a} is a carbon atom;
4) the antitumor agent described in any one of 1), 2) and 3), wherein the ring B^{a} is an optionally substituted benzene or pyridine;
5) the antitumor agent described in any one of 1) to 4), wherein the ring C^{a} is an optionally substituted pyrrole;
6) the antitumor agent described in 1), wherein the ring A^{a} is optionally substituted benzene or pyridine, the ring B^{a} is optionally substituted benzene, the ring C^{a} is optionally substituted pyrrole, W^{a} is a single bond, and Z^{a} is -NH-;
7) an antitumor agent comprising a sulfonamide-containing heterocyclic compound represented by the formula (I^{b}) : 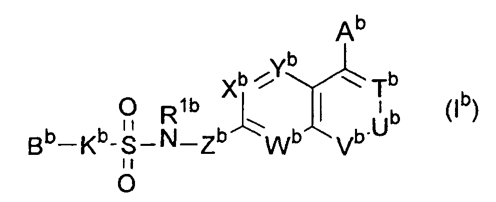 (wherein A^{b} represents a hydrogen atom, a halogen atom, a hydroxyl group, an alkyl or alkoxy group having one to four carbon atoms each of which may be substituted with a halogen atom, cyano group, - (CO) ₖ^{b}NR^{2b}R^{3b} (wherein R^{2b} and R^{3b} are the same as or different from each other and each represents a hydrogen atom or an alkyl group having one to four carbon atoms which may be substituted with a halogen atom, and k^{b} is 0 or 1) , an optionally substituted alkenyl or alkynyl group having two to four carbon atoms, or a phenyl or phenoxy group which may have a substituent selected from the group A below; B^{b} represents an aryl group or monocyclic heteroaryl group which may have a substituent selected from the group A below, or 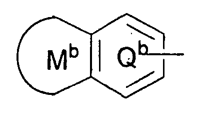 (wherein the ring Q^{b} represents an aromatic ring which may have one or two nitrogen atoms, and the ring M^{b} represents an unsaturated monocycle or heterocycle having five to twelve carbon atoms and having a double bond in common with the ring Q^{b}, and may have one to four hetero atoms selected from a nitrogen atom, an oxygen atom and a sulfur atom, the rings Q^{b} and M^{b} may have one nitrogen atom in common, and the rings Q^{b} and M^{b} may have a substituent selected from the group A below) ; K^{b} represents a single bond or - (CR^{4b}R^{5b}) m^{b}- (wherein R^{4b} and R^{5b} are the same as or different from each other and each represents a hydrogen atom and an alkyl group having one to four carbon atoms, and m^{b} is an integer of 1 or 2); T^{b}, W^{b}, X^{b} and Y^{b} are the same as or different from each other and each represents =C (D^{b}) - (wherein D^{b} represents a hydrogen atom, a halogen atom, hydroxyl group, an alkyl or alkoxy group having one to four carbon atoms each of which may be substituted with a halogen atom, cyano group, - (CO)ₙ^{b}NR^{6b}R^{7b} (wherein R^{6b} and R^{7b} are the same as or different from each other and each represents a hydrogen atom or an alkyl group having one to four carbon atoms which may be substituted with a halogen atom, and n^{b} is 0 or 1) or an optionally substituted alkenyl or alkynyl group having two or four carbon atoms; U^{b} and V^{b} are the same as or different from each other and each represents =C (D^{b})- (wherein D^{b} has the same meaning as defined above), a nitrogen atom, -CH₂-, an oxygen atom or -CO-; Z^{b} represents a single bond or -CO-NH-; R^{1b} represents a hydrogen atom or an alkyl group having one to four carbon atoms; and ------ represents a single bond or a double bond.
   group A: a halogen atom, hydroxyl group, an alkyl or alkoxy group having one to four carbon atoms which may be substituted with a halogen atom, cyano group, -R^{8b}R^{9b}N (NH)ₚ^{b}- (wherein R^{8b} and R^{9b} are the same as or different from each other and each represents a hydrogen atom or an alkyl group having one to four carbon atoms which may be substituted with a halogen atom, p^{b} is 0 or 1, and R^{8b} and R^{9b} may, together with nitrogen atoms to which they are bound, form a 5- or 6-membered ring, and the ring may further have nitrogen atom, oxygen atom or sulfur atom, and may have a substituent), an aminosulfonyl group which may be substituted with a mono- or dialkyl group having one to four carbon atoms, an optionally substituted acyl group having one to eight carbon atoms, a C1-C4 alkyl-S (O) ₛ^{b}-C1-C4 alkylene group (wherein s^{b} is an integer of 0, 1 or 2), a phenylsulfonylamino group which may have an alkyl having one to four carbon atoms or a substituent group, - (CO) _{q}^{b}NR^{10b}R^{11b} (wherein R^{10b} and R^{11b} are the same as or different from each other and each represents a hydrogen atom or an alkyl group having one to four carbon atoms which may be substituted with an amino group which may be substituted with a halogen atom or an alkyl group having one to four carbon atoms, and q^{b} is 0 or 1), or an optionally substituted aryl or heteroaryl group), a pharmacologically acceptable salt thereof or a hydrate of them combined with an angiogenesis inhibitor;
8) the antitumor agent described in 7), wherein U^{b} and V^{b} is =C (D^{b}) - (wherein D^{b} has the same meaning as defined above) or a nitrogen atom;
9) the antitumor agent described in 7) or 8), wherein Z^{b} is a single bond;
10) the antitumor agent described in any one of 7) to 9), wherein at least one of T^{b}, U^{b}, V^{b}, W^{b}, X^{b} and Y^{b} is a nitrogen atom;
11) the antitumor agent described in any one of 7) to 10) , wherein A^{b} represents a halogen atom; an alkyl or alkoxy group having one to four carbon atoms which may be substituted with a halogen atom; a cyano group; - (CO) ᵣ^{b}NR^{12b}R^{13b} whereupon R^{12b} and R^{13b} are the same or different from each other and each represent a hydrogen atom or an alkyl group having one to four carbon atoms which may be substituted with a halogen atom, and r^{b} is 0 or 1; or an optionally substituted alkenyl or alkynyl group having two to four carbon atoms;
12) the antitumor agent described in any one of 7) to 11), wherein only one of T^{b}, U^{b}, V^{b}, W^{b}, X^{b} and Y^{b} is a nitrogen atom;
13) the antitumor agent described in any one of 7) to 12), wherein only one of T^{b}, W^{b} and Y^{b} is a nitrogen atom;
14) an antitumor agent comprising a sulfonamide-containing heterocyclic compound represented by the formula (II): 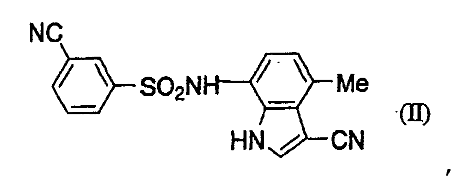 a pharmacologically acceptable salt thereof or a hydrate of them combined with an angiogenesis inhibitor;
15) the antitumor agent described in any one of 1) to 14), wherein the angiogenesis inhibitor is a VEGF receptor kinase inhibitor;
16) the antitumor agent described in 15) , wherein the VEGF receptor kinase inhibitor is ZD4190, ZD6474, SU5416, SU6668, SU11248, CEP-7055, CP-547,632, KNR663 or PTK787;
17) the antitumor agent described in 15), wherein the VEGF receptor kinase inhibitor is represented by the formula (III) : 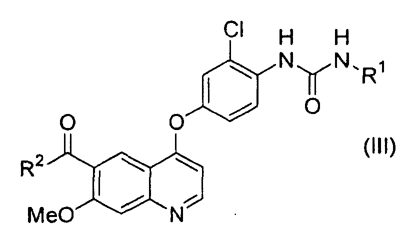 wherein R¹ represents a hydrogen atom, methyl group, ethyl group, n-propyl group or cyclopropyl group, and R² represents a group represented by the formula -NH₂ or a group represented by the formula -NHOMe;
18) the antitumor agent described in 15), wherein the VEGF receptor kinase inhibitor is represented by the formula (III) : 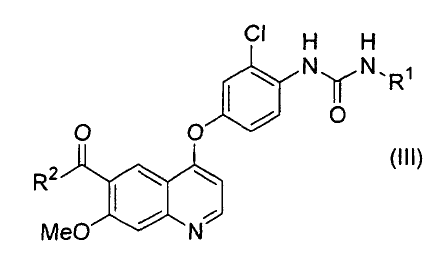 wherein R¹ represents ethyl or cyclopropyl group, and R² represents a group represented by the formula -NH₂ or a group represented by the formula -NHOMe;
19) the antitumor agent described in any one of 1) to 14) , wherein the angiogenesis inhibitor is a VEGF antibody;
20) the antitumor agent described in any one of 1) to 14), wherein the angiogenesis inhibitor is an FGF receptor kinase inhibitor;
21) the antitumor agent described in 20), wherein the FGF receptor kinase inhibitor is PD166866 or PD173074;
22) the antitumor agent described in any one of 1) to 14), wherein the angiogenesis inhibitor is an FGF antibody;
23) the antitumor agent described in any one of 1) to 22), wherein the tumor to be treated is malignant melanoma, malignant lymphoma, gastrointestinal cancer, pancreatic cancer, lung cancer, esophagus cancer, breast cancer, liver cancer, ovarian cancer, uterine cancer, prostate cancer, brain tumor, Kaposi's sarcoma, angioma, osteosarcoma or muscle sarcoma;
24) a kit for treating tumors, which comprises combining a preparation comprising asulfonamide-containing heterocyclic compound represented by the formula (I^{a}) : 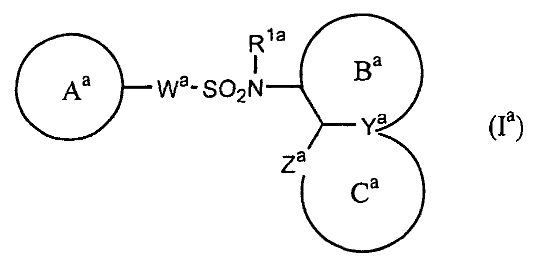 (wherein the ring A^{a} represents an optionally substituted monocyclic or bicyclic aromatic ring; the ring B^{a} represents a 6-membered unsaturated hydrocarbon or a unsaturated 6-membered heterocyclic ring containing one nitrogen atom as a heteroatom, each of which may be substituted; the ring C^{a} represents an optionally substituted 5-membered heterocyclic ring containing one or two nitrogen atoms; R^{1a} represents a hydrogen atom or an alkyl group having one to six carbon atoms; W^{a} represents a single bond or -CH=CH-; Y^{a} represents a carbon atom or a nitrogen atom; and Z^{a} represents -N (R^{2a}) - (wherein R^{2a} represents a hydrogen atomor a lower alkyl group) or a nitrogen atom) , a pharmacologically acceptable salt or a hydrate of them, and a preparation comprising an angiogenesis inhibitor;
25) the kit for treatment of tumors described in 24) , wherein W^{a} is a single bond;
26) the kit for treatment of tumors described in 25) , wherein W^{a} is a single bond, Z^{a} is -NH-, and Y^{a} is a carbon bond;
27) the kit for treatment of tumors described in any one of 24) , 25) and 26) , wherein the ring B^{a} is optionally substituted benzene or pyridine;
28) the kit for treatment of tumors described in any one of 24) to 27), wherein the ring C^{a} is optionally substituted pyrrole;
29) the kit for treating tumors described in 24), wherein the ring A^{a} is an optionally substituted benzene or pyridine, the ring B^{a} is an optionally substituted benzene, the ring C^{a} is an optionally substituted pyrrole, W^{a} is a single bond, and Z^{a} is -NH-;
30) a kit for treating tumors, which comprises combining a preparation comprising a sulfonamide-containing heterocyclic compound represented by the formula (I^{b}) : 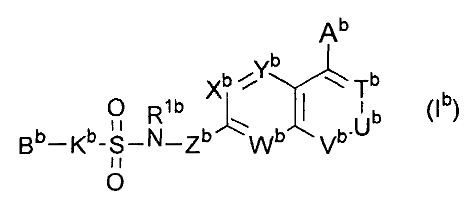 (wherein A^{b} represents a hydrogen atom, a halogen atom, a hydroxyl group, an alkyl or alkoxy group having one to four carbon atoms each of which may be substituted with a halogen atom, cyano group, - (CO)ₖ^{b}NR^{2b}R^{3b} (wherein R^{2b} and R^{3b} are the same as or different from each other and each represents a hydrogen atom or an alkyl group having one to four carbon atoms which may be substituted with a halogen atom, and k^{b} is 0 or 1), an optionally substituted alkenyl or alkynyl group having two to four carbon atoms, or a phenyl or phenoxy group which may have a substituent selected from the group A below; B^{b} represents an aryl group or monocyclic heteroaryl group which may have a substituent selected from the group A below, or 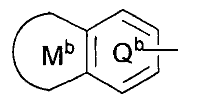 (wherein the ring Q^{b} represents an aromatic ring which may have one or two nitrogen atoms, and the ring M^{b} represents an unsaturated monocycle or heterocycle having five to twelve carbon atoms and having a double bond in common with the ring Q^{b}, and may have one to four hetero atoms selected from a nitrogen atom, an oxygen atom and a sulfur atom, the rings Q^{b} and M^{b} may have one nitrogen atom in common, and the rings Q^{b} and M^{b} may have a substituent selected from the group A below) ; K^{b} represents a single bond or - (CR^{4b}R^{5b})m^{b}- (wherein R^{4b} and R^{5b} are the same as or different from each other and each represents a hydrogen atom and an alkyl group having one to four carbon atoms, and m^{b} is an integer of 1 or 2); T^{b}, W^{b}, X^{b} and Y^{b} are the same as or different from each other and each represents =C (D^{b}) - (wherein D^{b} represents a hydrogen atom, a halogen atom, hydroxyl group, an alkyl or alkoxy group having one to four carbon atoms each of which may be substituted with a halogen atom, cyano group, - (CO) ₙ^{b}NR^{6b}R^{7b} (wherein R^{6b} and R^{7b} are the same as or different from each other and each represents a hydrogen atom or an alkyl group having one to four carbon atoms which may be substituted with a halogen atom, and n^{b} is 0 or 1) or an optionally substituted alkenyl or alkynyl group having two or four carbon atoms; U^{b} and V^{b} are the same as or different from each other and each represents =C (D^{b})- (wherein D^{b} has the same meaning as defined above), a nitrogen atom, -CH₂-, an oxygen atom or -CO-; Z^{b} represents a single bond or -CO-NH-; R^{1b} represents a hydrogen atom or an alkyl group having one to four carbon atoms; and ------ represents a single bond or a double bond.
   group A: a halogen atom, hydroxyl group, an alkyl or alkoxy group having one to four carbon atoms which may be substituted with a halogen atom, cyano group, -R^{8b}R^{9b}N (NH)ₚ^{b}- (wherein R^{8b} and R^{9b} are the same as or different from each other and each represents a hydrogen atom or an alkyl group having one to four carbon atoms which may be substituted with a halogen atom, p^{b} is 0 or 1, and R^{8b} and R^{9b} may, together with nitrogen atoms to which they are bound, form a 5- or 6-membered ring, and the ring may further have nitrogen atom, oxygen atom or sulfur atom, and may have a substituent), an aminosulfonyl group which may be substituted with a mono- or dialkyl group having one to four carbon atoms, an optionally substituted acyl group having one to eight carbon atoms, a C1-C4 alkyl-S (O) ₛ^{b}-C1-C4 alkylene group (wherein s^{b} is an integer of 0, 1 or 2), a phenylsulfonylamino group which may have an alkyl having one to four carbon atoms or a substituent group, -(CO)_{q}^{b}NR^{10b}R^{11b} (wherein R^{10b} and R^{11b} are the same as or different from each other and each represents a hydrogen atom or an alkyl group having one to four carbon atoms which may be substituted with an amino group which may be substituted with a halogen atom or an alkyl group having one to four carbon atoms, and q^{b} is 0 or 1) , or an optionally substituted aryl or heteroaryl group), a pharmacologically acceptable salt or a hydrate of them, and a preparation comprising an angiogenesis inhibitor;
31) the kit for treating tumors described in 30), wherein U^{b} and V^{b} is =C (D^{b}) - (wherein D^{b} has the same meaning as defined above) or a nitrogen atom;
32) the kit for treating tumors described in 30) or 31), wherein Z^{b} is a single bond;
33) the kit for treating tumors described in any one of 30) to 32), wherein at least one of T^{b}, U^{b}, V^{b}, W^{b}, X^{b} and Y^{b} is a nitrogen atom;
34) the kit for treating tumors described in any one of 30) to 33), wherein A^{b} represents a halogen atom, an alkyl or alkoxy group which may be substituted with a halogen atom, cyano group, - (CO)ᵣ^{b}NR^{12b}R^{13b} (wherein R^{12b} and R^{13b} are the same as or different from each other and each represents a halogen atom or an alkyl group having one to four carbon atoms which may be substituted with a halogen atom, and r^{b} is 0 or 1), or an optionally substituted alkenyl or alkynyl group having two to four carbon atoms;
35) the kit for treating tumors described in any one of 30) to 34), wherein only one of T^{b}, U^{b}, V^{b}, W^{b}, X^{b} and Y^{b} is a nitrogen atom;
36) the kit for treatment of tumors described in any one of claims 30) to 35), wherein only one of T^{b}, W^{b} and Y^{b} is a nitrogen atom;
37) a kit for treating tumors, which comprises a sulfonamide-containing heterocyclic compound represented by the formula (II) , a pharmacologically acceptable salt thereof or a hydrate of them combined with and an angiogenesis inhibitor; 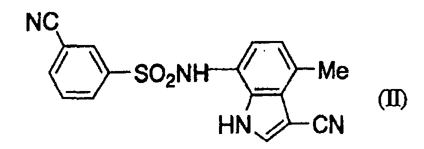
38) the kit for treating tumors described in any one of 24) to 37) , wherein the angiogenesis inhibitor is a VEGF receptor kinase inhibitor;
39) the kit for treating tumors described in 38), wherein the VEGF receptor kinase inhibitor is ZD4190, ZD6474, SU5416, SU6668, SU11248, CEP-7055, CP-547632, KNR663 or PTK787;
40) the kit for treating tumors described in 38), wherein the VEGF receptor kinase inhibitor is represented by the formula (III) : 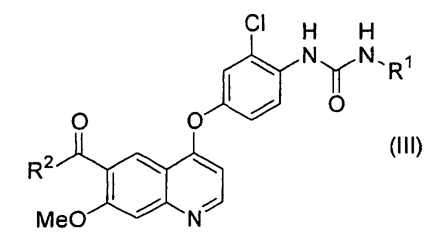 wherein R¹ represents a hydrogen atom, methyl group, ethyl group, n-propyl group or cyclopropyl group, and R² represents a group represented by the formula -NH₂ or a group represented by the formula -NHOMe;
41) The kit for treatment of tumors described in 38), wherein the VEGF receptor kinase inhibitor is represented by the formula (III) : 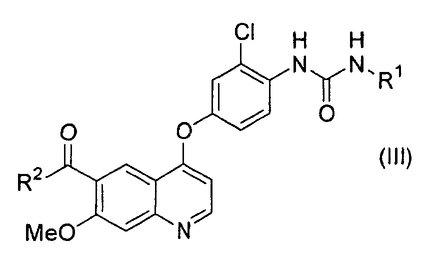 wherein R¹ represents ethyl or cyclopropyl group, and R² represents a group represented by the formula -NH₂ or a group represented by the formula -NHOMe;
42) the kit for treating tumors described in any one of 24) to 37), wherein the angiogenesis inhibitor is a VEGF antibody;
43) the kit for treating tumors described in any one of 24) to 37), wherein the angiogenesis inhibitor is an FGF receptor kinase inhibitor;
44) the kit for treating tumors described in 43), wherein the FGF receptor kinase inhibitor is PD166866 or PD173074;
45) the kit for treating tumors described in any one of 24) to 37), wherein the angiogenesis inhibitor is an FGF antibody;
46) the kit for treating tumors described in any one of 24) to 45), wherein the tumor to be treated is malignant melanoma, malignant lymphoma, gastrointestinal cancer, pancreatic cancer, lung cancer, esophagus cancer, breast cancer, liver cancer, ovarian cancer, uterine cancer, prostate cancer, brain tumor, Kaposi's sarcoma, angioma, osteosarcoma or muscle sarcoma;
47) the kit described in 24) , which comprises administering the two preparations simultaneously or separately after a predetermined time;
48) a method of treating cancers, which comprises administering the compound described in 14), a pharmacologically acceptable salt thereof or a hydrate of them, and one angiogenesis inhibitor selected from a VEGF receptor kinase inhibitor, a VEGF antibody, an FGF receptor kinase inhibitor and an FDF antibody to a patient simultaneously or separately after a predetermined time;
49) use of the compound described in 14), a pharmacologically acceptable salt thereof or a hydrate of them, and one angiogenesis inhibitor selected from a VEGF receptor kinase inhibitor, a VEGF antibody, an FGF receptor kinase inhibitor and an FDF antibody for treating cancers, which comprises administering them simultaneously or separately after a predetermined time; and
50) use of the compound described in 14), apharmacologically acceptable salt thereof or a hydrate of them, and one angiogenesis inhibitor selected from a VEGF receptor kinase inhibitor, a VEGF antibody, an FGF receptor kinase inhibitor and an FDF antibody, for producing a combined preparation.

Hereinafter, embodiments of the invention are described.

In the formula (I^{a}), the "optionally substituted monocyclic or bicyclic aromatic ring" represented by the ring A^{a} is an aromatic hydrocarbon or an aromatic heterocyclic ring containing at least one of a nitrogen atom, an oxygen atom and a sulfur atom, and the ring may have one to three substituents thereon. Examples of main aromatic rings contained in the ring A^{a} include pyrrole, pyrazole, imidazole, thiophene, furan, thiazole, oxazole, benzene, pyridine, pyrimidine, pyrazine, pyridazine, naphthalene, quinoline, isoquinoline, phthalazine, naphthyridine, quinoxaline, quinazoline, cinnoline, indole, isoindole, indolizine, indazole, benzofuran, benzothiophene, benzoxazole, benzimidazole, benzopyrazole, benzothiazoleetc. The aromatic ring may have one to three substituents, and when there are a plurality of substituents, they may be the same or different. The substituent includes, for example, an amino group which may be substituted with a lower alkyl group or a lower cycloalkyl group, a lower alkyl group, a lower alkoxy group, a hydroxyl group, a nitro group, a mercapto group, a cyano group, a lower alkylthio group, a halogen group, a group represented by the formula -a^{a}-b^{b}- (wherein a^{a} represents a single bond, - (CH₂) ₖ^{a}-, -O- (CH₂) ₖ^{a}-, -S- (CH₂) ₖ^{a}- or -N (R^{3a}) -(CH₂) ₖ^{a}-, k^{a} is an integer of 1 to 5, R^{3a} represents a hydrogen atom or a lower alkyl group, b^{a} represents -CH₂-d^{a} (wherein d^{a} represents an amino group which may be substituted with a lower alkyl group, or a halogen atom, a hydroxyl group, a lower alkylthio group, a cyano group or a lower alkoxy group)), a group represented by the formula -a^{a}-e^{a}-f^{a} (wherein a^{a} has the same meaning as defined above, e^{a} represents -S(O)- or -S(O)₂-, f^{a} represents an amino group which may be substituted with a lower alkyl or lower alkoxy group, a lower alkyl group, a trifluoromethyl group, - (CH₂)ₘ^{a} -b^{a} or -N (R^{4a}) - (CH₂)ₘ^{a}-b^{a} (wherein b^{a} has the same meaning as defined above, R^{4a} represents a hydrogen atom or a lower alkyl group, and m^{a} is an integer of 1 to 5)), a group represented by the formula -a^{a}-g^{a}-h^{a} (wherein a^{a} has the same meaning as defined above, g^{a} represents -C(O)- or -C (S) -, and h^{a} represents an amino group which may be substituted with a lower alkyl group, or a hydroxyl group, a lower alkyl group, a lower alkoxy group, or -(CH₂)ₙ^{a}-b^{a} or -N (R^{5a}) - (CH₂)ₙ^{a}-b^{a} (wherein b^{a} has the same meaning as defined above, R^{5a} represents a hydrogen atom or a lower alkyl group, and n^{a} is an integer of 1 to 5)), a group represented by the formula -a^{a}-N (R^{6a}) -g^{a}-i^{a} (a^{a} and g^{a} have the same meanings as defined above, R^{6a} represents a hydrogen atom or a lower alkyl group, and i^{a} represents a hydrogen atom, a lower alkoxy group or a group represented by f^{a} (f^{a} has the same meaning as defined above) ) , a group represented by the formula - a^{a}-N (R^{7a}) -e^{a}-f^{a} (wherein a^{a}, e^{a} and f^{a} have the same meanings as defined above, and R^{7a} represents a hydrogen atom or a lower alkyl group), a group represented by the formula -(CH₂)ₚ^{a}-j^{a}-(CH₂)_{q}^{a}-b^{a} (wherein j^{a} represents an oxygen atom or a sulfur atom, b^{a} has the same meaning as defined above, and p^{a} and q^{a} are the same as or different from each other and each represents an integer of 1 to 5), a group represented by the formula - (CH₂)ᵤ^{a}-Ar^{a} (wherein Ar^{a} represents a phenyl or heteroaryl group which may be substituted a lower alkyl group, a lower alkoxy group or a halogen atom, and u^{a} is 0 or an integer of 1 to 5), the formula -CONH- (CH₂)ᵤ^{a}-Ar^{a} (wherein Ar^{a} and u^{a} have the same meanings as defined above) or the formula - SO₂- (CH₂)ᵤ^{a}-Ar^{a} (wherein Ar^{a} and u^{a} have the same meanings as defined above).

In the above examples of the substituent, when the amino group is substituted with two alkyl groups, these alkyl groups may be bound to each other to form a 5 or 6-membered ring. When the ring A^{a} is a nitrogen-containing heterocyclic ring having a hydroxyl or mercapto group, these groups may have a resonance structure in the form of an oxo or thioxo group.

The "optionally substituted, 6-membered unsaturated hydrocarbon or unsaturated 6-membered heterocyclic ring containing one nitrogen atom as a heteroatom" represented by the ring B^{a} is optionally partially hydrogenated benzene or pyridine, and the ring may have one or two substituents thereon, and when there are two substituents, they may be the same or different.

The "optionally substituted, 5-membered heterocyclic ring containing one or two nitrogen atoms" represented by the ring C^{a} is optionally partially hydrogenated pyrrole, pyrazole or imidazole, and the ring may have one or two substituents thereon, and when there are two substituents, they may be the same or different.

The substituents which may be possessed by the rings B^{a} and C^{a} include, for example, a halogen group, a cyano group, a lower alkyl group, a lower alkoxy group, a hydroxyl group, an oxo group, the formula -C(O)-r^{a} (wherein r^{a} represents a hydrogen atom, an amino group which may be substituted with a lower alkyl group, a lower alkyl group, a lower alkoxy group or a hydroxyl group), an amino group which may be substituted with a lower alkyl group, trifluoromethyl group, etc.

In the formula (I^{a}) , the lower alkyl group represented by R^{1a} and R^{2a} or defined as a substituent which may be possessed by the rings A^{a}, B^{a} and C^{a} refers to a linear or branched alkyl group having 1 to 6 carbon atoms. For example, methyl group, ethyl group, n-propyl group, isopropyl group, n-butyl group, isobutyl group, sec-butyl group, tert-butyl group, n-pentyl group (amyl group), isopentyl group, neopentyl group, tert-pentyl group, 1-methylbutyl group, 2-methylbutyl group, 1,2-dimethylpropyl group, n-hexyl group, isohexyl group, 1-methylpentyl group, 2-methylpentyl group, 3-methylpentyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,1,2-trimethylpropyl group, 1,2,2-trimethylpropyl group, 1-ethyl-1-methylpropyl group and 1-ethyl-2-methylpropyl group may be proposed. Among these groups, preferable groups include methyl group, ethyl group, n-propyl group, isopropyl group, n-butyl group and isobutyl group, and the most preferable group includes methyl group, ethyl group, n-propyl group and isopropyl group.

The lower cycloalkyl group defined as a substituent which may possessed by the ring A^{a} includes cyclopropyl group, cyclopentyl group and cyclohexyl group.

The lower alkoxy group defined as a substituent which may possessed by the rings A^{a}, B^{a} and C^{a} refers to lower alkoxy groups derived from the above-mentioned lower alkyl groups, and includes methoxy group, ethoxy group, n-propoxy group, isopropoxy group, n-butoxy group, isobutoxy group and tert-butoxy group. Among these, the most preferable group includes methoxy group and ethoxy group. The halogen atom includes a fluorine atom, chlorine atom, bromine atom etc.

Preferable among these compounds are:
1) N-(3-cyano-4-methyl-1H-indol-7-yl)-3-cyanobenzenesulfonamide,
2) N-(3-cyano-4-methyl-1H-indol-7-yl)-6-chloro-3-pyridinesulfonamide,
3) N-(3-bromo-5-methyl-1H-indol-7-yl)-4-sulfamoylbenzenesulfonamide,
4) N-(5-bromo-3-chloro-1H-indol-7-yl)-6-amino-3-pyridinesulfonamide,
5) N-(3-bromo-5-methyl-1H-indol-7-yl)-3-cyanobenzenesulfonamide,
6) N-(4-bromo-1H-indol-7-yl)-4-cyanobenzenesulfonamide,
7) N-(4-chloro-1H-indol-7-yl)-6-amino-3-pyridinesulfonamide,
8) N-(3-bromo-4-chloro-1H-indol-7-yl)-6-amino-3-pyridinesulfonamide,
9) N-(3-bromo-5-methyl-1H-indol-7-yl)-5-cyano-2-thiophenesulfonamide,
10) N-(4-bromo-3-chloro-1H-indol-7-yl)-2-amino-5-pyrimidinesulfonamide, and
11) N-(3-chloro-1H-indol-7-yl)-4-sulfamoylbenzenesulfonamide.

The sulfonamide derivative represented by the formula (I^{a}) may form a salt with an acid or base. The present invention also encompasses salts of the compound (I^{a}) . The salt with an acid includes, for example, inorganic salts such as hydrochloride, hydrobromate or sulfate, and salts with organic acids such as acetic acid, lactic acid, succinic acid, fumaric acid, maleic acid, citric acid, benzoic acid, methanesulfonic acid or p-toluenesulfonic acid. The salt with a base includes inorganic salts such as sodium salt, potassium salt or calcium salt, as well as salts with organic bases such as triethylamine, arginine or lysine.

In the present invention, the "aromatic ring which may have one or two nitrogen atoms" represented by the ring Q^{b} is an aromatic hydrocarbon or a 6-membered aromatic heterocyclic ring containing one or two nitrogen atoms. Examples of main aromatic rings contained in the ring Q^{b} include benzene, pyridine, pyrimidine, pyrazine, pyridazine etc. The ring M in the phrase "ring M represents an unsaturated monocycle or heterocycle having five to twelve carbon atoms, and may have one to four heteroatoms selected from a nitrogen atom, an oxygen atom and a sulfur atom" is an unsaturated monocycle or heterocycle having a double bond in common with the ring Q^{b}, and includes aromatic hydrocarbons such as benzene, naphthalene etc., unsaturated hydrocarbons such as cyclopentene, cyclohexene, cycloheptene, cyclooctene, cyclopentadiene, cycloheptadiene, cyclooctadiene etc., and unsaturated heterocyclic rings such as tetrahydropyridine, pyrrole, furan, thiophene, oxazole, isoxazole, thiazole, isothiazole, pyrazole, imidazole, triazole, pyridine, pyrimidine, pyrazine, pyridazine, triazine, indole, isoindole, quinoline, isoquinoline, indazolidine, naphthyridine, benzofuran, benzopyran, benzothiophene, benzimidazole, benzoxazole, benzothiazole, pyrolopyridine, pyridopyrimidine, imidazopyridine etc. The phrase "rings Q^{b} and M^{b} may have one nitrogen atom in common" means that a nitrogen atom is present at a condensation site of the two rings, and the ring thus formed includes indazolidine, imidazo[1,2-a]pyridine, imidazo[1,5-a]pyridine, pyrazolo[1,5-a]pyrimidine etc.

In the present invention, the alkyl group having one to four carbon atoms in R^{1b}, R^{4b} and R^{5b}, or the alkyl group having one to four carbon atoms which may be substituted with a halogen atom in A^{b}, D^{b}, R^{1b}, R^{2b}, R^{3b}, R^{6b}, R^{7b}, R^{8b}, R^{9b}, R^{10b}, R^{11b}, R^{12b}, R^{13b}, R^{14b}, R^{15b}, G^{1b}, G^{2b} and the group A refers to a linear or branched alkyl group having one to four carbon atoms. For example, methyl group, ethyl group, n-propyl group, isopropyl group, n-butyl group, isobutyl group, sec-butyl group and tert-butyl group may be proposed. The phrase "which may be substituted with a halogen atom" means that such alkyl group may be substituted with a halogen atom selected from a fluorine atom, a chlorine atom, a bromine atom and an iodine atom. For example, monofluoromethyl group, monochloromethyl group, difluoromethyl group, trifluoromethyl group, 1- or 2-monofluoroethyl group, 1- or 2-monochloroethyl group, 1- or 2-monobromoethyl group, 1,2-difluoroethyl group, 1,2-dichloroethyl group, 1,1,2,2,2-pentafluoroethyl group and 3,3,3-trifluoropropyl group may be proposed. Preferable examples of these groups include monofluoromethyl group, difluoromethyl group, trifluoromethyl group, 1- or 2-monofluoroethyl group, 1,2-difluoroethyl group and 1,1,2,2,2-pentafluoroethyl group.

In the present invention, the alkoxy group having one to four carbon atoms in the phrase "the alkoxy group having one to four carbon atoms which may be substituted with a halogen atom" in A^{b}, D^{b} and the group A refers to a linear or branched alkoxy group having one to four carbon atoms. For example, methoxy group, ethoxy group, n-propyloxy group, isopropyloxy group, n-butyloxy group, isobutyloxy group, sec-butyloxy group and tert-butyloxy group may be proposed. The phrase "which may be substituted with a halogen atom" means that such alkoxy group may be substituted with a halogen atom selected from a fluorine atom, a chlorine atom, a bromine atom and an iodine atom. For example, monofluoromethoxy group, difluoromethoxy group, trifluoromethoxy group, 1- or 2-monofluoroethoxy group, 1- or 2-monochloroethoxy group, 1- or 2-monobromoethoxy group, 1,2-difluoroethoxy group, 1,1,2,2,2-pentafluoroethoxy group and 3,3,3-trifluoropropyloxy group may be proposed. Preferable examples of these groups include monofluoromethoxy group, difluoromethoxy group, trifluoromethoxy group, 1- or 2-monofluoroethoxy group, 1,2-difluoroethoxy group and 1,1,2,2,2-pentafluoroethoxy group.

In the present invention, the alkenyl or alkynyl group having two to four carbon atom in A^{b} and D^{b} refers to an alkenyl or alkynyl group having two to four carbon atoms, such as vinyl group, allyl group, 2-or3-butenyl group, 1, 3-butadienyl group, ethynyl group, 2-propynyl group, 2-methylethynyl group, 2- or 3-butynyl group etc.

In the present invention, the aryl group in B^{b} and the group A refers to an aromatic hydrocarbon such as phenyl group, naphthyl group etc. The heteroaryl group is a monocycle or heterocycle containing one or more nitrogen atoms, oxygen atoms or sulfur atoms. For example, pyrrolyl, imidazolyl group, pyrazolyl group, triazolyl group, furyl group, thienyl group, oxazolyl group, isoxazolyl group, thiazolyl group, isothiazolyl group, thiadiazolyl group, pyridyl group, pyrimidyl group, pyrazyl group, indolyl group, indolizinyl group, benzoimidazolyl group, benzothiazolyl group, benzoxazolyl group, quinolinyl group, isoquinolinyl group, quinazolinyl group, phthalazinyl group etc. may be proposed.

In the present invention, the phrase "R^{8b} and R^{9b} may, together with nitrogen atoms to which they are bound, form a 5 or 6-membered ring, and the ring may further have a nitrogen atom, an oxygen atom or a sulfur atom" in R^{8b} and R^{9b} means that R^{8b} and R^{9b} may, together with nitrogen atoms to which they are bound, form a pyrrolidinyl group, piperidinyl group, morpholino group, thiomorpholino group, piperazinyl group etc.

In the "aminosulfonyl group which may be substituted with a mono or dialkyl group having one to four carbon atoms", the "C1-C4 alkyl-S(O)ₛ^{b}-C1-C4 alkylene group", the "phenylsulfonylamino group which may have an alkyl group having one to four carbon atoms or a substituent group" and the "alkyl group having one to four carbon atoms which may be substituted with an alkyl group having one to four carbon atoms" in the group A in the present invention, the "alkyl group" means the same alkyl group as described above, and the "alkylene group" includes methylene group, ethylene group, propylene group, butylene group, methylmethylene group, 1- or 2-methylethylene group, 1-, 2- or 3-methylpropylene group and dimethylmethylene group.

The above groups further mean an alkanoyl group having one to eight carbon atoms, formyl group, acetyl group, propionyl group, butyryl group, isobutyryl group, valeryl group, benzoyl group etc.

In the "amino group which may have a protecting group" in J^{b} in the present invention, the protecting group may be any group known as a protecting group for an amino group in usual organic synthesis, and is not particularly limited. For example, benzyloxycarbonyl group, t-butoxycarbonyl group, formyl group, acetyl group, chloroacetyl group, 2,2,2-trichloroethyl group, benzylidene group, benzhydryl group, trityl group etc. may be proposed. The protecting group in the carboxyl group which may have a protecting group, and the protecting group for the carboxy group in R^{16b} may be any group known as a protecting group for a carboxyl group in usual organic synthesis, and is not particularly limited. For example, methyl group, ethyl group, propyl group, isopropyl group, t-butyl group, methoxymethyl group, 2,2,2-trichloroethyl group, pivaloyloxymethyl group, benzyl group etc. may be proposed.

In the present invention, the substituent in the "optionally substituted" includes the above defined halogen atom, alkyl or alkoxy group having one to four carbon atoms, each of which may be substituted with a halogen atom, hydroxy group, hydroxyalkyl group having one to four carbon atoms, amino group which may be substituted with a mono or dialkyl group having one to four carbon group, alkenyl or alkynyl group having two to four carbon atoms, cyano group, acyl group having one to eight carbon atoms, aminosulfonyl group which may be substituted with a mono or dialkyl group having one to four carbon groups, carboxy group, alkoxycarbonyl group having one to four carbon atoms, carbamoyl group which may be substituted with a mono or dialkyl group having one to four carbon atoms, etc.

The sulfonamide-containing heterocyclic compound represented by the formula (I^{b}) may form a salt with an acid or base. The present invention also encompasses salts of the compound (I^{b}). The salt with an acid includes, for example, inorganic salts such as hydrochloride, hydrobromate or sulfate, and salts with organic acids such as acetic acid, lactic acid, succinic acid, fumaric acid, maleic acid, citric acid, benzoic acid, methanesulfonicacidorp-toluenesulfonicacid. The salt with a base includes inorganic salts such as sodium salt, potassium salt or calcium salt, as well as salts with organic bases such as triethylamine, arginine or lysine.

As a matter of course, the present invention encompasses hydrates of these compounds and any possible optical isomers thereof if any. The present invention further encompasses compounds exhibiting an angiogenesis inhibitory action, which are formed in vivo from the present compound by metabolism such as oxidation, reduction, hydrolysis and conjugation. Further, the present invention encompasses compounds undergoing metabolism such as oxidation, reduction, hydrolysis and conjugation *in vivo* to form the compound of the invention.

The compound (I^{a}) according to the present invention can be produced by various methods. For example, some methods are described specifically in JP-A 7-165708 and JP-A 8-231505.

In the present invention, the angiogenesis inhibitor is not particularly limited insofar as it is different in site of action from the sulfonamide-containing heterocyclic compound, but the angiogenesis inhibitor is preferably a VEGF inhibitor or FGF inhibitor, more preferably a VEGF receptor kinase inhibitor, a VEGF antibody (Cancer Res., 55, 5296-5301, 1995), an FGF receptor kinase inhibitor or an FGF antibody (Cancer Res., 51, 6180-4, 1991).

The VEGF receptor kinase inhibitor is more preferably ZD4190 (Cancer Res., 60, 970-975, 2000), ZD6474 (Proc. Am. Assoc. Cancer Res., 42, 583, 2001), SU5416 (Cancer Res., 59, 99-106, 1999), SU6668 (Cancer Res., 60, 4152-4160, 2000), SU11248 (Clinical Cancer Res., 9, 327-337, 2003) , CEP-7055 (Proc. Am. Assoc. Cancer Res., 43, 1080, 2002), CP-547632 (Proc. Am. Soc. Clin. Oncolo. 21, (Abstract 16) , 2002) , KRN633 (Proc. Am. Assoc. Cancer Res., 43, 175, 2002) or PTK787 (Cancer Res., 60, 2179-2189, 2000) (see formula IV).

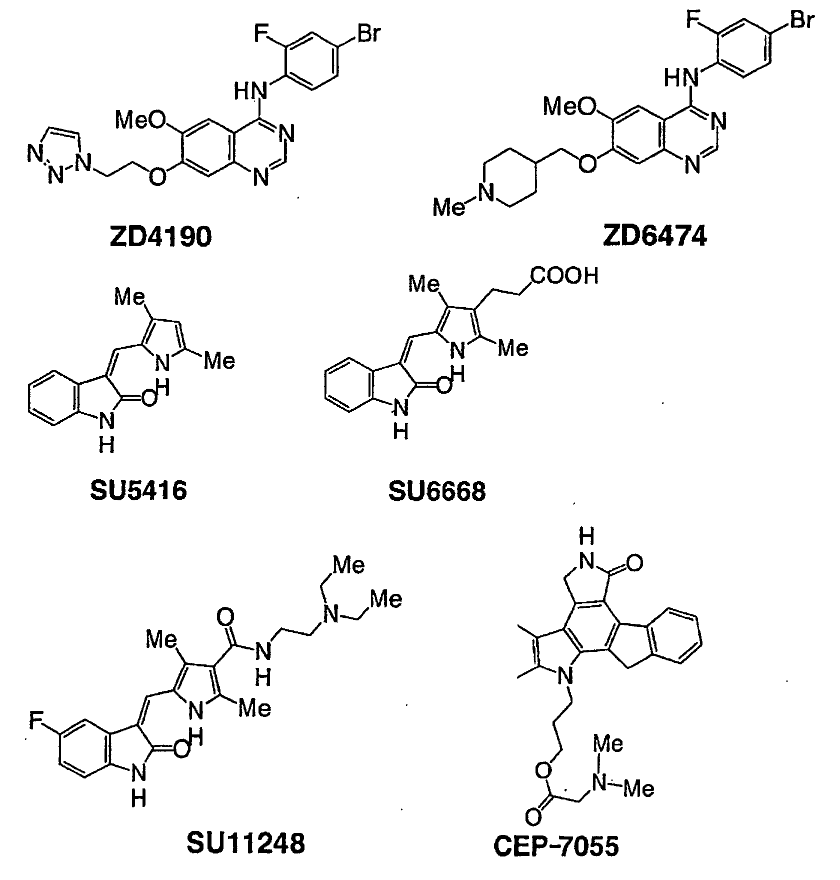

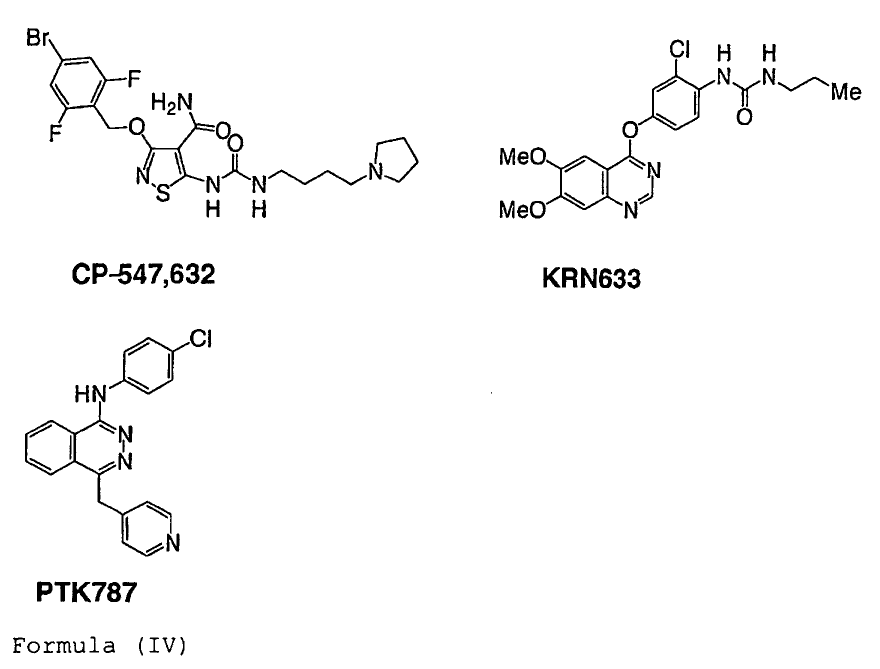

The FGF receptor kinase inhibitor is more preferably PD166866 (J. M. C., 40, 2296-2303, 1997) or PD173074 (EMBO J., 17, 5896-5904, 1998).

When the compound of the invention is used as a pharmaceutical preparation, it is administered orally or parenterally. The dosage of the sulfonamide-containing heterocyclic compound of the invention is not particularly limited, and is varied depending on severity of the symptom, age, sex, weight and sensitivity of a patient, administration method, administration period, administration frequency, the properties, formulation and type of a pharmaceutical preparation, and the type of the active ingredient, but the dosage is usually 10 to 6000 mg, preferably about 50 to 4000 mg, more preferably 100 to 3000mg, which is administered usually into an adult man in one to three divided portions every day.

The dosage of the VEGF receptor kinase inhibitor in the present invention is not particularly limited, but is usually 10 to 6000 mg, preferably about 50 to 4000 mg, more preferably 50 to 2000 mg, which is administered into an adult man usually in one to three divided portions every day.

The dosage of the FGF receptor kinase inhibitor in the present invention is not particularly limited, but is usually 10 to 6000 mg, preferably about 50 to 4000 mg, more preferably 50 to 2000 mg, which is administered into an adult man usually in one to three divided portions every day.

The dosage of the VEGF antibody in the present invention is not particularly limited, but is usually 1 to 6000 mg, preferably about 10 to 2000 mg, more preferably 10 to 1000 mg, which is administered usually once at an interval of 1 day to 1 week.

The dosage of the FGF antibody in the present invention is not particularly limited, but is usually 1 to 6000 mg, preferably about 10 to 2000 mg, more preferably 10 to 1000 mg, which is administered usually once at an interval of 1 day to 1 week.

The amount of the sulfonamide-containing heterocyclic compound used is varied depending on the combination thereof with the VEGF receptor kinase inhibitor or FGF receptor kinase inhibitor, but is usually about 0.01- to 100-fold (ratio by weight), more preferably 0.1- to 10-fold (ratio by weight), based on the VEGF receptor kinase inhibitor or FGF receptor kinase inhibitor.

When an oral solid preparation is produced, the active ingredient is blended with ordinarily used fillers and if necessary with a binder, a disintegrating agent, a lubricant, a coloring agent, a flavoring agent and then formed in a usual manner into tablets, coated tablets, granules, fine granules powder, capsules etc.

The filers include e.g. lactose, corn starch, white sugar, glucose, sorbitol, crystalline cellulose, silicon dioxide etc.; the binder includes e.g. polyvinyl alcohol, ethyl cellulose, methyl cellulose, arabic gum, hydroxypropyl cellulose, hydroxypropylmethyl cellulose etc.; the lubricant includes e.g. magnesium stearate, talc, silica etc.; the coloring agent includes e.g. those coloring agents approved to be added to pharmaceutical preparations; and the flavoring agent includes cocoa powder, menthol, aromatic powder, peppermint oil, borneol, cinnamon powder etc. The tablets and granules may be coated with a sugar or gelatin coating or if necessary with another suitable coating.

When an injection is prepared, the active ingredient is blended if necessary with a pH adjuster, a buffer, a suspension agent, a solubilizer, a stabilizer, an isotonizing agent, a preservative etc. and then formed in a usual manner into an intravenous, subcutaneous or intramuscular injection. If necessary, the liquid preparation may be formed into a freeze-dried product.

The suspension agent includes, for example, methyl cellulose, Polysorbate 80, hydroxyethyl cellulose, arabic gum, tragacanth powder, carboxymethyl cellulose sodium, polyoxyethylene sorbitan monolaurate etc.

The solubilizer includes, for example, polyoxyethylene hardened castor oil, Polysorbate 80, nicotinic acid amide, polyoxyethylene sorbitan monolaurate, Macrogol, castor oil fatty ethyl ester etc.

The stabilizer includes, for example, sodium sulfite, sodium metasulfite, ether etc., and the preservative includes, for example, methyl p-oxybenzoate, ethyl p-oxybenzoate, sorbic acid, phenol, cresol, chlorocresol, etc.

According to the present invention, the angiogenesis inhibitor can be combined effectively for use in treatment of cancers.

### Brief Description of the Drawing

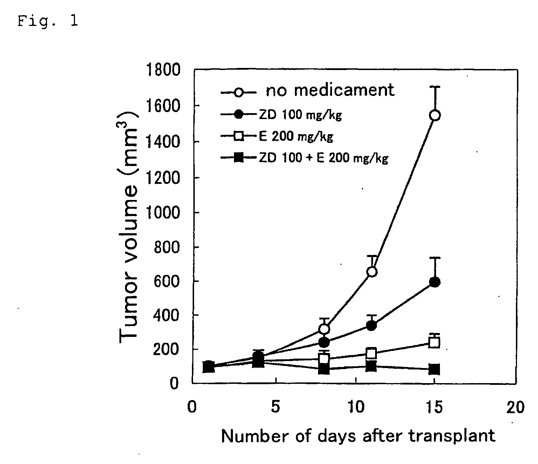

Fig. 1 shows the synergistic effect of E7820 and ZD6474 in a mouse model with grown cancer.

### Examples

Hereinafter, the invention is described in more detail by reference to the Examples, but the invention is not limited thereto.

### Example 1 Synergistic effect of VEGF inhibitor and E7820 on proliferation of VEGF-induced endothelial cells

Human umbilical vein endothelial cells were suspended at a density of 1.5×10⁴ cells/ml in human endothelial-SFM basal growth medium (GIBCO BRL) containing 20 ng/ml VEGF (Wako Pure Chemical Industries, Ltd.) and 2% FCS, and 100 µl of this suspension was added to each well in a 96-well plate and cultured at 37°C in a CO₂ incubator. On the next day, 1/2 serial dilutions of VEGF receptor kinase inhibitors (ZD6474, ZD4190, SU5416, SU6668) or VEGFantibody as VEGF inhibitors, 1/2 serial dilutions of VEGF antibody, 1/2 serial dilutions of E7820, and dilutions containing two of the above substances were prepared respectively, and each dilution was added in a volume of 100 µl/well to each well containing the cultured human umbilical vein endothelial cells, and the cells were further cultured.

After 3 days, 5 ml cell counting kit solution (Wako Pure Chemical Industries, Ltd.) were diluted with PBS to give 40 ml dilution, and 50 µl of this dilution were added to each well, then incubated at 37°C for 3 to 4 hours and measured for its absorbance at 415 nm by a plate reader (Corona Denki Co., Ltd.) . The synergistic effect was calculated according to a formula of Chou et al. (Adv. Enzyme Regul., 22, 27-55, 1984).

The combination index of E7820 and the VEGF receptor kinase inhibitor or VEGF antibody was 1 or less, indicating that there was a synergistic effect therebetween.

**Table 1**

| Synergistic effect of E7820 and ZD4190, ZD6474, SU5416, SU6668 or VEGF antibody | | | |
|---|---|---|---|
| VEGF inhibitor | Inhibition rate (fa) | Combination index (Cl) | Combination effect |
| ZD6474 | 0.5 | 0.4 | Synergistic |
| SU5416 | 0.5 | 0.4 | Synergistic |
| SU6668 | 0.5 | 0.5 | Synergistic |
| VEGF antibody | 0.5 | 0.8 | Synergistic |
| ZD4190 | 0.5 | 0.3 | Synergistic |

### Example 2 Synergistic effect of FGF inhibitor and E7820 on proliferation of FGF-induced endothelial cells

Human umbilical vein endothelial cells were suspended at a density of 1×10⁴ cells/ml in human endothelial-SFM basal growth medium (GIBCO BRL) containing 20 ng/ml bFGF (GIBCO BRL) and 2% FCS, and 100 µl of this suspension were added to each well in a 96-well plate and cultured at 37°C in a CO₂ incubator. On the next day, 1/2 serial dilutions of FGF receptor kinase inhibitors (PD166866, PD173074) or FGF antibody as FGF inhibitors, 1/2 serial dilutions of E7820, and dilutions containing two of the above substances were prepared respectively, and each dilution was added in a volume of 100 µl/well to each well containing the cultured human umbilical vein endothelial cells, and the cells were further cultured.

After 3 days, 5 ml cell counting kit solution (Wako Pure Chemical Industries, Ltd.) were diluted with PBS to give 40 ml dilution, and 50 µl of this dilution were added to each well, then incubated at 37°C for 3 to 4 hours and measured for its absorbance at 415 nm by a plate reader (Corona Denki Co., Ltd.) . The synergistic effect was calculated according to a formula of Chou et al. (Adv. Enzyme Regul., 22, 27-55, 1984).

The combination index of E7820 and the FGF receptor kinase inhibitor or FGF antibody was 1 or less, indicating that there was a synergistic effect therebetween.

**Table 2**

| Synergistic effect of E7820 and PD166866, PD173074 or FGF antibody | | | |
|---|---|---|---|
| FGF inhibitor | Inhibition rate (fa) | Combination Index (Cl) | Combination effect |
| PD166866 | 0.5 | 0.5 | Synergistic |
| PD173074 | 0.5 | 0.4 | Synergistic |
| FGF antibody | 0.5 | 0.5 | Synergistic |

### Example 3 Synergistic effect of VEGF or FGF inhibitor (ZD6474, VEGF antibody, FGF antibody) and E7820 on tube formation of endothelial cell

400 µl of type I collagen gel (Nitta Gelatin) were added onto each well of a 24-well plate (FALCON) and gelled at 37°C in a CO₂ incubator for 40 min. A serum-free medium (human endothelial-SFM basal growth medium; GIBCO BRL) containing 20 ng/ml EGF (GIBCO BRL) was prepared, and 200 µl of this medium solution were added onto each well containing the type I collagen gel. Human umbilical vein endothelial cells (HUVEC) were suspended in a serum-free medium (human endothelial-SFM basal growth medium; GIBCO BRL) to prepare a cell suspension at a density of 5×10⁵ cells/ml. 200 µl of this suspension were put into each well containing the type I collagen gel and serum-free medium, and cultured overnight.

On the next day, 400 µl type I collagen gel were laid thereon and gelled at 37°C in a CO₂ incubator for 3 hours, and 1.5 ml serum-free medium containing VEGF or FGF inhibitor (ZD6474, VEGF antibody, PD166866, FGF antibody), both E7820 and VEGF, or both FGF inhibitor and E7820 (the VEGF inhibitor well had contained 10 ng/ml EGF and 20 ng/ml VEGF (Wako Pure Chemical Industries, Ltd.), and the FGF inhibitor well had contained 10 ng/ml EGF and 20 ng/ml bFGF (GIBCO BRL) ) were added thereto, and the cells were further cultured at 37°C in a CO₂ incubator for 3 days.

After incubation, 400 µl of 3. 3 mg/ml MTT (SIGMA) were added into each well and reacted at 37°C in a CO₂ incubator for 3 hours to stain the cells. Under a microscope (SZX12 manufactured by OLYMPUS) , an image of a tube formed by HUVEC was incorporated (M-3204C manufactured by OLYMPUS). The tube length in the incorporated image was measured by image analysis software Mac SCOPE 2.56 (manufactured by MITANI) to quantify the tube formation of HUVEC. The synergistic effect was calculated according to a formula of Chou et al. (Adv. Enzyme Regul., 22, 27-55, 1984).

The combination index of E7820 and VEGF receptor kinase inhibitor, VEGF antibody, PD166866 or FGF antibody was 1 or less, indicating their synergistic effect.

**Table 3**

| Synergistic effect of E7820 and ZD6474, VEGF antibody or FGF antibody | | | |
|---|---|---|---|
| Inhibitor | Inhibition rate (fa) | Combination index (Cl) | Combination effect |
| ZD6474 | 0.5 | 0.4 | Synergistic |
| VEGF antibody | 0.5 | 0.7 | Synergistic |
| PD166866 | 0.5 | 0.6 | Synergistic |
| FGF antibody | 0.5 | 0.6 | Synergistic |

### Example 4 Combination effect in a mouse model with grown cancer.

1×10⁷ KP-1 or Colo320DM cells/0.1 ml PBS/mouse were inoculated subcutaneously to mice (KSN Slc, female). From 1 to 2 weeks after the transplantation, 0.5% aqueous methyl cellulose solution, E7820 (0.5% methyl cellulose suspension: 50, 100, 200mg/kg: twice a day) , or ZD6474 (0. 5% methyl cellulose suspension: 50, 100 mg/kg: once a day), or both of E7820 (50, 100, 200 mg/kg: twice a day) and ZD6474 (50, 100 mg/kg: once a day) , were orally administered for 2 weeks. The transplanted tumor volume was measured every day, and the relative tumor volume was calculated according to the following equation: Relative tumor volume=tumor volume on the day after last administration/tumor volume at first administration

The antitumor effect was expressed in T/C % (T/C % = relative tumor volume in the chemical administration group/relative tumor volume in the control group). The combination effect was calculated according to a formula of Takahashi et al. (Cancer Res., 61, 7846-7854, 2001).

As shown in Fig. 1, tumor growth was inhibited by E7820 or ZD6474 singly, and excellent antitumor effect was demonstrated by combination. As shown in Table 4, the combination effect of two compounds was higher than an expected value of additive effect, thus indicating that the combined effect was regarded as synergistic effect.

**Table 4.**

| Synergistic effect of E7820 and ZD6474 in a mouse model with grown cancer. | | | | |
|---|---|---|---|---|
| Tumor | E7820 single (T/C %) | ZD6474 single (T/C%) | Expected value | Combination use of E7820·ZD6474 (T/C %) |
| KP-1 | 83% | 47% | 39% | 29% |
| Colo320DM | 60 % | 56% | 34% | 28% |

## Claims

1. An antitumor agent comprising a sulfonamide-containing heterocyclic compound represented by the formula (I^{a})_{:} 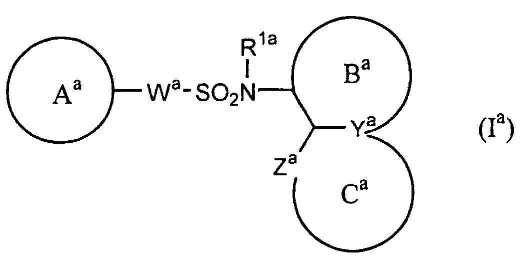 (wherein, the ring A^{a} represents an optionally substituted monocyclic or bicyclic aromatic ring; the ring B^{a} represents a 6-membered unsaturated hydrocarbon or a unsaturated 6-membered heterocyclic ring containing one nitrogen atom as a heteroatom, each of which may be substituted; the ring C^{a} represents an optionally substituted 5-membered heterocyclic ring containing one or two nitrogen atoms; R^{1a} represents a hydrogen atom or an alkyl group having one to six carbon atoms; W^{a} represents a single bond or -CH=CH-; Y^{a} represents a carbon atom or a nitrogen atom; and Z^{a} represents -N(R^{2a})- (wherein R^{2a} represents a hydrogen atom or a lower alkyl group) or a nitrogen atom) , a pharmacologically acceptable salt thereof or a hydrate of them combined with an angiogenesis inhibitor.

2. The antitumor agent according to claim 1, wherein W^{a} is a single bond.

3. The antitumor agent according to claim 1, wherein W^{a} is a single bond, Z^{a} is -NH- and Y^{a} is a carbon atom.

4. The antitumor agent according to any one of claims 1, 2 and 3, wherein the ring B^{a} is an optionally substituted benzene or pyridine.

5. The antitumor agent according to any one of claims 1 to 4, wherein the ring C^{a} is an optionally substituted pyrrole.

6. The antitumor agent according to claim 1, wherein the ring A^{a} is an optionally substituted benzene or pyridine, the ring B^{a} is an optionally substituted benzene, the ring C^{a} is an optionally substituted pyrrole, W^{a} is a single bond, and Z^{a} is -NH-.

7. An antitumor agent comprising a sulfonamide-containing heterocyclic compound represented by the formula (I^{b}) : 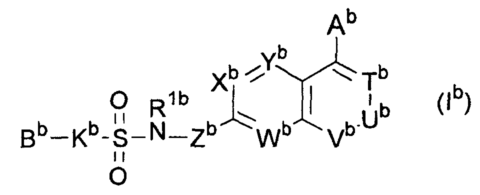 (wherein A^{b} represents a hydrogen atom, a halogen atom, a hydroxyl group, an alkyl or alkoxy group having one to four carbon atoms each of which may be substituted with a halogen atom, cyano group, - (CO)ₖ^{b}NR^{2b}R^{3b} (wherein R^{2b} and R^{3b} are the same as or different from each other and each represents a hydrogen atom or an alkyl group having one to four carbon atoms which may be substituted with a halogen atom, and k^{b} is 0 or 1) , an optionally substituted alkenyl or alkynyl group having two to four carbon atoms, or a phenyl or phenoxy group which may have a substituent selected from the group A below; B^{b} represents an aryl group or monocyclic heteroaryl group which may have a substituent selected from the group A below, or 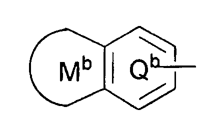 (wherein the ring Q^{b} represents an aromatic ring which may have one or two nitrogen atoms, and the ring M^{b} represents an unsaturated monocycle or heterocycle having five to twelve carbon atoms and having a double bond in common with the ring Q^{b}, and may have one to four hetero atoms selected from a nitrogen atom, an oxygen atom and a sulfur atom, the rings Q^{b} and M^{b} may have one nitrogen atom in common, and the rings Q^{b} and M^{b} may have a substituent selected fromthe groupAbelow) ; K^{b} represents a single bond or -(CR^{4b}R^{5b})m^{b}- (wherein R^{4b} and R^{5b} are the same as or different from each other and each represents a hydrogen atom and an alkyl group having one to four carbon atoms, and m^{b} is an integer of 1 or 2); T^{b}, W^{b}, X^{b} and Y^{b} are the same as or different from each other and each represents =C (D^{b}) - (wherein D^{b} represents a hydrogen atom, a halogen atom, hydroxyl group, an alkyl or alkoxy group having one to four carbon atoms each of which may be substituted with a halogen atom, cyano group, - (CO)ₙ^{b}NR^{6b}R^{7b} (wherein R^{6b} and R^{7b} are the same as or different from each other and each represents a hydrogen atom or an alkyl group having one to four carbon atoms which may be substituted with a halogen atom, and n^{b} is 0 or 1) or an optionally substituted alkenyl or alkynyl group having two or four carbon atoms; U^{b} and V^{b} are the same as or different from each other and each represents =C (D^{b})- (wherein D^{b} has the same meaning as defined above), a nitrogen atom, -CH₂-, an oxygen atom or -CO-; Z^{b} represents a single bond or -CO-NH-; R^{1b} represents a hydrogen atom or an alkyl group having one to four carbon atoms; and ------ represents a single bond or a double bond.
group A: a halogen atom, hydroxyl group, an alkyl or alkoxy group having one to four carbon atoms which may be substituted with a halogen atom, cyano group, -R^{8b}R^{9b}N (NH)ₚ^{b}- (wherein R^{8b} and R^{9b} are the same as or different from each other and each represents a hydrogen atom or an alkyl group having one to four carbon atoms which may be substituted with a halogen atom, p^{b} is 0 or 1, and R^{8b} and R^{9b} may, together with nitrogen atoms to which they are bound, form a 5- or 6-membered ring, and the ring may further have nitrogen atom, oxygen atom or sulfur atom, and may have a substituent), an aminosulfonyl group which may be substituted with a mono- or dialkyl group having one to four carbon atoms, an optionally substituted acyl group having one to eight carbon atoms, a C1-C4 alkyl-S (O) ₛ^{b}-C1-C4 alkylene group (wherein s^{b} is an integer of 0, 1 or 2), a phenylsulfonylamino group which may have an alkyl having one to four carbon atoms or a substituent group, -(CO)_{q}^{b}NR^{10b}R^{11b} (wherein R^{10b} and R^{11b} are the same as or different from each other and each represents a hydrogen atom or an alkyl group having one to four carbon atoms which may be substituted with an amino group which may be substituted with a halogen atom or an alkyl group having one to four carbon atoms, and q^{b} is 0 or 1) , or an optionally substituted aryl or heteroaryl group), a pharmacologically acceptable salt thereof or a hydrate of them combined with an angiogenesis inhibitor.

8. The antitumor agent according to claim 7, wherein U^{b} and V^{b} is =C (D^{b})- (wherein D^{b} has the same meaning as defined above) or a nitrogen atom.

9. The antitumor agent according to claim 7 or 8, wherein Z^{b} is a single bond.

10. The antitumor agent according to any one of claims 7 to 9, wherein at least one of T^{b}, U^{b}, V^{b}, W^{b}, X^{b} and Y^{b} is a nitrogen atom.

11. The antitumor agent according to any one of claims 7 to 10, wherein A^{b} represents a halogen atom; an alkyl or alkoxy group having one to four carbon atoms, each of which may be substituted with a halogen atom; a cyano group; - (CO) ᵣ ^{b}NR^{12b}R^{13b} (wherein R^{12b} and R^{13b} are the same as or different from each other and each represents a hydrogen atom or an alkyl group having one to four carbon atoms which may be substituted with a halogen atom, and r^{b} is 0 or 1) ; or an optionally substituted alkenyl or alkynyl group having two to four carbon atoms.

12. The antitumor agent according to any one of claims 7 to 11, wherein only one of T^{b}, U^{b}, V^{b}, W^{b}, X^{b} and Y^{b} is a nitrogen atom.

13. The antitumor agent according to any one of claims 7 to 12, wherein only one of T^{b}, W^{b} and Y^{b} is a nitrogen atom.

14. An antitumor agent comprising a sulfonamide-containing heterocyclic compound represented by the formula (II), a pharmacologically acceptable salt thereof or a hydrate of them combined with an angiogenesis inhibitor.

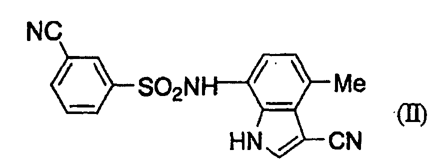

15. The antitumor agent according to any one of claims 1 to 14, wherein the angiogenesis inhibitor is a VEGF receptor kinase inhibitor.

16. The antitumor agent according to claim 15, wherein the VEGF receptor kinase inhibitor is ZD4190, ZD6474, SU5416, SU6668, SU11248, CEP-7055, CP-547632, KNR663 or PTK787.

17. The antitumor agent according to claim 15, wherein the VEGF receptor kinase inhibitor is represented by the formula (III): 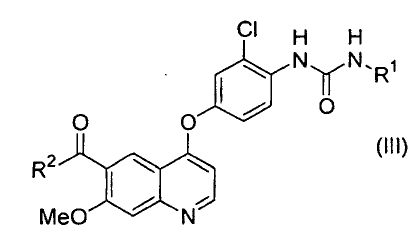 wherein R¹ represents a hydrogen atom, methyl group, ethyl group, n-propyl group or cyclopropyl group, and R² represents a group represented by the formula -NH₂ or a group represented by the formula -NHOMe.

18. The antitumor agent according to claim 15, wherein the VEGF receptor kinase inhibitor is represented by the formula (III) : 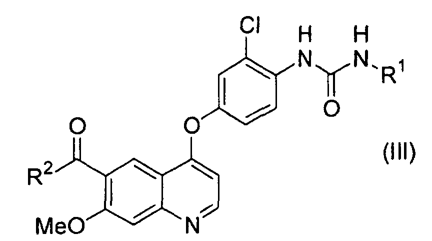 wherein R¹ represents ethyl or cyclopropyl group, and R² represents a group represented by the formula -NH₂ or a group represented by the formula -NHOMe.

19. The antitumor agent according to any one of claims 1 to 14, wherein the angiogenesis inhibitor is a VEGF antibody.

20. The antitumor agent according to any one of claims 1 to 14, wherein the angiogenesis inhibitor is an FGF receptor kinase inhibitor.

21. The antitumor agent according to claim 20, wherein the FGF receptor kinase inhibitor is PD166866 or PD173074.

22. The antitumor agent according to any one of claims 1 to 14, wherein the angiogenesis inhibitor is an FGF antibody.

23. The antitumor agent according to any one of claims 1 to 22, wherein the tumor to be treated is malignant melanoma, malignant lymphoma, gastrointestinal cancer, pancreatic cancer, lung cancer, esophagus cancer, breast cancer, liver cancer, ovarian cancer, uterine cancer, prostate cancer, brain tumor, Kaposi's sarcoma, angioma, osteosarcoma or muscle sarcoma.

24. A kit for treating tumors, which comprises combining a preparation comprising a sulfonamide-containing heterocyclic compound represented by the formula (I^{a}): 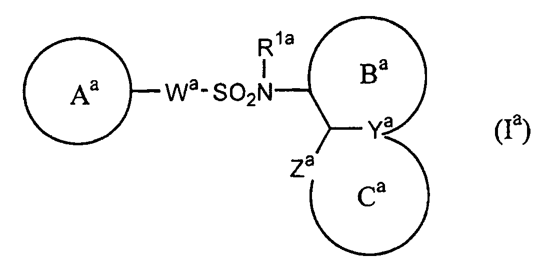 (wherein the ring A^{a} represents an optionally substituted monocyclic or bicyclic aromatic ring; the ring B^{a} represents a 6-membered unsaturated hydrocarbon or a unsaturated 6-membered heterocyclic ring containing one nitrogen atom as a heteroatom, each of which may be substituted; the ring C^{a} represents an optionally substituted 5-membered heterocyclic ring containing one or two nitrogen atoms; R^{1a} represents a hydrogen atom or an alkyl group having one to six carbon atoms; W^{a} represents a single bond or -CH=CH-; Y^{a} represents a carbon atom or a nitrogen atom; and Z^{a} represents -N(R^{2a})- (wherein R^{2a} represents a hydrogen atom or a lower alkyl group) or a nitrogen atom) , a pharmacologically acceptable salt or a hydrate of them, and a preparation comprising an angiogenesis inhibitor.

25. The kit for treating tumors according to claim 24, wherein W^{a} is a single bond.

26. The kit for treating tumors according to claim 25, wherein W^{a} is a single bond, Z^{a} is -NH-, and Y^{a} is a carbon atom.

27. The kit for treating tumors according to any one of claims 24, 25 and 26, wherein the ring B^{a} is an optionally substituted benzene or pyridine.

28. The kit for treating tumors according to any one of claims 24 to 27, wherein the ring C^{a} is an optionally substituted pyrrole.

29. The kit for treating tumors according to claim 24, wherein the ring A^{a} is an optionally substituted benzene or pyridine, the ring B^{a} is an optionally substituted benzene, the ring C^{a} is an optionally substituted pyrrole, W^{a} is a single bond, and Z^{a} is -NH-.

30. A kit for treating tumors, which comprises combining a preparation comprising a sulfonamide-containing heterocyclic compound represented by the formula (I^{b}): 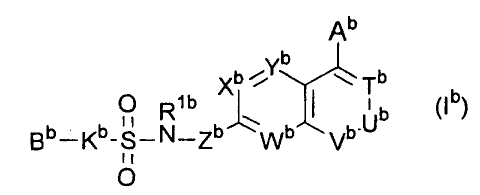 (wherein A^{b} represents a hydrogen atom, a halogen atom, a hydroxyl group, an alkyl or alkoxy group having one to four carbon atoms each of which may be substituted with a halogen atom, cyano group, - (CO) ₖ^{b}NR^{2b}R^{3b} (wherein R^{2b} and R^{3b} are the same as or different from each other and each represents a hydrogen atom or an alkyl group having one to four carbon atoms which may be substituted with a halogen atom, and k^{b} is 0 or 1) , an optionally substituted alkenyl or alkynyl group having two to four carbon atoms, or a phenyl or phenoxy group which may have a substituent selected from the group A below; B^{b} represents an aryl group or monocyclic heteroaryl group which may have a substituent selected from the group A below, or 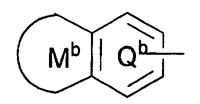 (wherein the ring Q^{b} represents an aromatic ring which may have one or two nitrogen atoms, and the ring M^{b} represents an unsaturated monocycle or heterocycle having five to twelve carbon atoms and having a double bond in common with the ring Q^{b}, and may have one to four hetero atoms selected from a nitrogen atom, an oxygen atom and a sulfur atom, the rings Q^{b} and M^{b} may have one nitrogen atom in common, and the rings Q^{b} and M^{b} may have a substituent selected from the group A below) ; K^{b} represents a single bond or -(CR^{4b}R^{5b})m^{b}- (wherein R^{4b} and R^{5b} are the same as or different from each other and each represents a hydrogen atom and an alkyl group having one to four carbon atoms, and m^{b} is an integer of 1 or 2); T^{b}, W^{b}, X^{b} and Y^{b} are the same as or different from each other and each represents =C ( D^{b}) - (wherein D^{b} represents a hydrogen atom, a halogen atom, hydroxyl group, an alkyl or alkoxy group having one to four carbon atoms each of which may be substituted with a halogen atom, cyano group, - (CO)ₙ^{b}NR^{6b}R^{7b} (wherein R^{6b} and R^{7b} are the same as or different from each other and each represents a hydrogen atom or an alkyl group having one to four carbon atoms which may be substituted with a halogen atom, and n^{b} is 0 or 1) or an optionally substituted alkenyl or alkynyl group having two or four carbon atoms; U^{b} and V^{b} are the same as or different from each other and each represents =C (D^{b}) - (wherein D^{b} has the same meaning as defined above), a nitrogen atom, -CH₂-, an oxygen atom or -CO-; Z^{b} represents a single bond or -CO-NH-; R^{1b} represents a hydrogen atom or an alkyl group having one to four carbon atoms; and ------ represents a single bond or a double bond.
group A: a halogen atom, hydroxyl group, an alkyl or alkoxy group having one to four carbon atoms which may be substituted with a halogen atom, cyano group, -R^{8b}R^{9b}N (NH)ₚ^{b}- (wherein R^{8b} and R^{9b} are the same as or different from each other and each represents a hydrogen atom or an alkyl group having one to four carbon atoms which may be substituted with a halogen atom, p^{b} is 0 or 1, and R^{8b} and R^{9b} may, together with nitrogen atoms to which they are bound, form a 5- or 6-membered ring, and the ring may further have nitrogen atom, oxygen atom or sulfur atom, and may have a substituent), an aminosulfonyl group which may be substituted with a mono- or dialkyl group having one to four carbon atoms, an optionally substituted acyl group having one to eight carbon atoms, a C1-C4 alkyl-S (O) ₛ^{b}-C1-C4 alkylene group (wherein s^{b} is an integer of 0, 1 or 2), a phenylsulfonylamino group which may have an alkyl having one to four carbon atoms or a substituent group, - (CO) _{q}^{b}NR^{10b}R^{11b} (wherein R^{10b} and R^{11b} are the same as or different from each other and each represents a hydrogen atom or an alkyl group having one to four carbon atoms which may be substituted with an amino group which may be substituted with a halogen atom or an alkyl group having one to four carbon atoms, and q^{b} is 0 or 1), or an optionally substituted aryl or heteroaryl group), a pharmacologically acceptable salt or a hydrate of them, and a preparation comprising an angiogenesis inhibitor.

31. The kit for treating tumors according to claim 30, wherein U^{b} and V^{b} is =C(D^{b}) - (wherein D^{b} has the same meaning as defined above) or a nitrogen atom.

32. The kit for treating tumors according to claim 30 or 31, wherein Z^{b} is a single bond.

33. The kit for treating tumors according to any one of claims 30 to 32, wherein at least one of T^{b}, U^{b}, V^{b}, W^{b}, X^{b} and Y^{b} is a nitrogen atom.

34. The kit for treating tumors according to any one of claims 30 to 33, wherein A^{b} represents a halogen atom, an alkyl or alkoxy group which may be substituted with a halogen atom, cyano group, - (CO)ᵣ^{b} NR^{12b}R^{13b} (wherein R^{12b} and R^{13b} are the same as or different from each other and each represents a halogen atom or an alkyl group having one to four carbon atoms which may be substituted with a halogen atom, and r^{b} is 0 or 1), or an optionally substituted alkenyl or alkynyl group having two to four carbon atoms.

35. The kit for treating tumors according to any one of claims 30 to 34, wherein only one of T^{b}, U^{b}, V^{b}, W^{b}, X^{b} and Y^{b} is a nitrogen atom.

36. The kit for treatment of tumors according to any one of claims 30 to 35, wherein only one of T^{b}, W^{b} and Y^{b} is a nitrogen atom.

37. A kit for treating tumors, which comprises a sulfonamide-containing heterocyclic compound represented by the formula (II), a pharmacologically acceptable salt thereof or a hydrate of them combined with and an angiogenesis inhibitor.

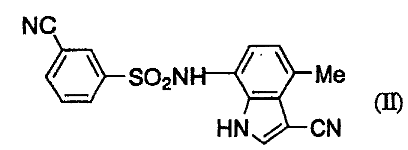

38. The kit for treating tumors according to any one of claims 24 to 37, wherein the angiogenesis inhibitor is a VEGF receptor kinase inhibitor.

39. The kit for treating tumors according to claim 38, wherein the VEGF receptor kinase inhibitor is ZD4190, ZD6474, SU5416, SU6668, SU11248, CEP-7055, CP-547632, KNR663 or PTK787.

40. The kit for treating tumors according to claim 38, wherein the VEGF receptor kinase inhibitor is represented by the formula (III): 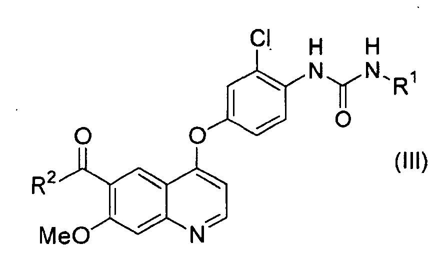 wherein R¹ represents a hydrogen atom, methyl group, ethyl group, n-propyl group or cyclopropyl group, and R² represents a group represented by the formula -NH₂ or a group represented by the formula -NHOMe.

41. The kit for treatment of tumors according to claim 38, wherein the VEGF receptor kinase inhibitor is represented by the formula (III): 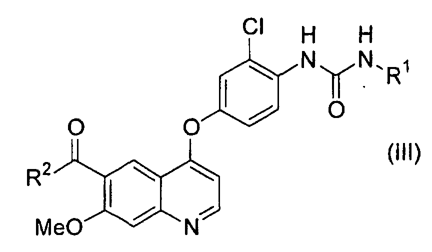 wherein R¹ represents ethyl or cyclopropyl group, and R² represents a group represented by the formula -NH₂ or a group represented by the formula -NHOMe.

42. The kit for treating tumors according to any one of claims 24 to 37, wherein the angiogenesis inhibitor is a VEGF antibody.

43. The kit for treating tumors according to any one of claims 24 to 37, wherein the angiogenesis inhibitor is an FGF receptor kinase inhibitor.

44. The kit for treating tumors according to claim 43, wherein the FGF receptor kinase inhibitor is PD166866 or PD173074.

45. The kit for treating tumors according to any one of claims 24 to 37, wherein the angiogenesis inhibitor is an FGF antibody.

46. The kit for treating tumors according to any one of claims 24 to 45, wherein the tumor to be treated is malignant melanoma, malignant lymphoma, gastrointestinal cancer, pancreatic cancer, lung cancer, esophagus cancer, breast cancer, liver cancer, ovarian cancer, uterine cancer, prostate cancer, brain tumor, Kaposi's sarcoma, angioma, osteosarcoma or muscle sarcoma.

47. The kit according to claim 24, which comprises administering the two preparations simultaneously or separately after a predetermined time.

48. A method of treating cancers, which comprises administering the compound described in claim 14, a pharmacologically acceptable salt thereof or a hydrate of them, and one angiogenesis inhibitor selected from a VEGF receptor kinase inhibitor, a VEGF antibody, an FGF receptor kinase inhibitor and an FDF antibody to a patient simultaneously or separately after a predetermined time.

49. Use of the compound described in claim 14, a pharmacologically acceptable salt thereof or a hydrate of them, and one angiogenesis inhibitor selected from a VEGF receptor kinase inhibitor, a VEGF antibody, an FGF receptor kinase inhibitor and an FDF antibody for treating cancers, which comprises administering them simultaneously or separately after a predetermined time.

50. Use of the compound described in claim 14, a pharmacologically acceptable salt thereof or a hydrate of them, and one angiogenesis inhibitor selected from a VEGF receptor kinase inhibitor, a VEGF antibody, an FGF receptor kinase inhibitor and an FDF antibody, for producing a combined preparation.
